# EUROPÄISCHE PATENTSCHRIFT

(11) **EP 3 592 210 B1**
(45) Veröffentlichungstag und Bekanntmachung des Hinweises auf die Patenterteilung: **20.01.2021**
(21) Anmeldenummer: 18708965.1
(22) Anmeldetag: 28.02.2018
(51) Int. Cl.: G01B 9/02

(54) **OPTISCHE SONDE UND VERFAHREN ZUM BETRIEB DER OPTISCHEN SONDE**
OPTICAL PROBE AND METHOD FOR OPERATING THE OPTICAL PROBE
SONDE OPTIQUE ET PROCÉDÉ POUR LE FONCTIONNEMENT DE LA SONDE OPTIQUE

(30) Priorität: 06.03.2017 DE 102017104617
(43) Veröffentlichungstag der Anmeldung: 15.01.2020
(73) Patentinhaber: GRINTECH GmbH, 07745 Jena (DE)
(72) Erfinder: MESSERSCHMIDT, Bernhard, 07749 Jena (DE); MATZ, Gregor, 07743 Jena (DE)
(74) Vertreter: Epping - Hermann - Fischer
(86) Internationale Anmeldenummer: PCT/EP2018/054970
(87) Internationale Veröffentlichungsnummer: WO 2018/162303

(56) Entgegenhaltungen:
- DE-A1-102006 046 554
- US-A1- 2008 058 629
- US-A1- 2015 049 339
- M. BLATTMANN ET AL: "Bimodal endoscopic probe combining white-light microscopy and optical coherence tomography", APPLIED OPTICS, Bd. 55, Nr. 15, 20. Mai 2016 (2016-05-20), Seite 4261, XP055471307, WASHINGTON, DC; US ISSN: 0003-6935, DOI: 10.1364/AO.55.004261
- Jessica Mavadia ET AL: "An all-fiber-optic endoscopy platform for simultaneous OCT and fluorescence imaging", Biomedical optics express, 1. November 2012 (2012-11-01), Seiten 2851-609, XP55430847, United States DOI: 10.1364/BOE.3.002851 Gefunden im Internet: URL:https://pdfs.semanticscholar.org/0dfc/ 1cb84456a459eef864d4d4deb0ed4bad908e.pdf

## Beschreibung

Die Erfindung betrifft eine optische Sonde, insbesondere eine miniaturisierte bildgebende optische Sonde, die beispielsweise für die Untersuchung von Gewebe in der Biologie oder Medizin geeignet ist.

Miniaturisierte bildgebende Sonden, die an ein zu untersuchendes Objekt herangeführt werden können, sind vor allem in der biologischen Forschung oder in der Medizin von Interesse, um minimal-invasive Untersuchungen zu ermöglichen oder lebendes Gewebe unter normalen Lebensumständen über längere Zeiträume zu beobachten.

Verschiedene miniaturisierte bildgebende Sonden sind in den folgenden Dokumenten beschrieben: US2015/0049339 A1, US2008/0058629 A1, DE10 2006 046 554 A1, so wie in den Artikeln "Bimodal endoscopic probe combining white-light microscopy and optical coherence tomography", M. Blattmann et al., Appl. Optics Vol. 55, No. 15, S. 4261-4269 und "An all-fiber-optic endoscopy platform for simultaneous OCT and fluorescence imaging", J. Mavadia et al., Biomedical Optics Express Vol. 3, no. 11, S. 2851-2859.

Besonderes Potenzial besitzen die optische Kohärenztomographie (OCT, optical coherence tomography) für die 3-dimensionale Bildgebung der Morphologie von zum Beispiel mehreren Millimeter dicken Gewebeschichten, die Raman-Spektroskopie für die Identifizierung molekularchemischer Bestandteile des Gewebes, die Multiphotonen-Fluoreszenzmikroskopie für eine hochaufgelöste submikrometergenaue Unterscheidung chemischer Gewebebestandteile (insbesondere endogener Fluorophore wie NADPH und Flavinen sowie exogener Chromophore), die Frequenzverdoppelung (SHG, second harmonic generation) um regelmäßig angeordnete Zellbestandteilen wie Collagen, Mikrotubuli und Myosin zu lokalisieren, die Mikroskopie basierend auf kohärenter Anti-Stokes Raman-Streuung (CARS, Coherent Anti-Stokes Raman Scattering) bzw. Mikroskopie basierend auf stimulierter Raman Streuung (SRS, stimulated Raman scattering) für eine molekular-chemisch differenzierende Bildgebung.

Der Erfindung liegt gemäß zumindest einem Aspekt die Aufgabe zugrunde, eine miniaturisierte optische Sonde und ein Verfahren zum Betrieb der optischen Sonde anzugeben, wobei die Sonde biologische oder medizinische Untersuchungen mit weiter verbesserter Sensitivität ermöglicht.

Gemäß zumindest einer Ausgestaltung umfasst die optische Sonde ein rückseitiges Ende zum Anschluss an eine Steuereinheit und ein frontseitiges Ende zur optischen Untersuchung eines Objekts. An dem frontseitigen Ende der Sonde ist insbesondere eine Lichtdurchtrittsöffnung vorgesehen, durch die Beleuchtungslicht aus der Sonde auf ein Objekt wie zum Beispiel Gewebe bei biologischen oder medizinischen Untersuchungen gerichtet werden kann. Weiterhin kann durch die Lichtdurchtrittsöffnung vom Objekt zurückkehrendes Objektlicht, das durch Wechselwirkung des Beleuchtungslichts mit dem Objekt entsteht, wieder in die Sonde eintreten. An dem rückseitigen Ende kann die Sonde mit einer Verbindungsleitung optisch und elektronisch an eine Steuervorrichtung angeschlossen sein, die insbesondere zur Ansteuerung der Sonde und zur Signalauswertung dient.

Die Sonde enthält vorteilhaft einen ersten optischen Strahlengang für ein scannendes bildgebendes Verfahren und einen zweiten optischen Strahlengang für ein spektroskopisches Verfahren. Die Sonde ist insbesondere dazu vorgesehen, ein Objekt mit zwei verschiedenen optischen Verfahren zu untersuchen, wobei zumindest eines der Verfahren ein bildgebendes Verfahren ist, bei dem durch einen Scanvorgang ein zwei- oder dreidimensionaler Bereich des Objekts optisch abgetastet wird. Das zweite Verfahren ist vorteilhaft ein spektroskopisches Verfahren, besonders bevorzugt konfokale Raman-Spektroskopie. Die optische Sonde ist somit eine multimodale Sonde, wobei mit zwei optischen Strahlengängen in der Sonde vorteilhaft die Untersuchung eines Objekts mit einem bildgebenden Verfahren und einem spektroskopischen Verfahren ermöglicht wird. Insbesondere kann vorgesehen sein, dass an einer zu untersuchenden Stelle eines Objekts abwechselnd eine Untersuchung mit dem bildgebenden Verfahren und mit dem spektroskopischen Verfahren erfolgt. Die Sensitivität und Spezifität eines mit der Sonde durchführbaren Untersuchungsverfahrens wird auf diese Weise vorteilhaft erhöht.

Die optische Sonde ermöglicht insbesondere die Kombination eines bildgebenden Verfahrens wie OCT, MultiphotonenMikroskopie, konfokale Einzelphotonen-Fluoreszenz - oder Reflexionsmikroskopie oder CARS/SRS-Mikroskopie, bei dem punktförmig fokussiert die Objektebene durch ein geeignetes Anregungs- bzw. Beleuchtungslicht abgerastert wird, mit einer konfokalen Anordnung einer Raman-Anregung und Detektion des inelastisch gestreuten Spektrums. Da das ins langwellige verschobene Raman-Signal sehr schwach ist und nur einen ca. 10⁻¹²-ten Anteil der Anregungsintensität betragen kann, ist eine ortsaufgelöste Raman-Bildgebung für medizinische und biologische Anwendungen an lebenden Geweben oder Organen nicht sinnvoll, so dass ein Raman-Signal vorzugsweise nur punktuell bzw. integral über einen begrenzten Anregungsfleck, vorzugsweise im Zentrum des Bildfeldes der bildgebenden Sonde, aufgenommen wird.

Gemäß der Erfindung umfasst die Sonde eine erste Lichtleitfaser in dem ersten optischen Strahlengang, die dazu eingerichtet ist, ein Beleuchtungslicht für das scannende bildgebende Verfahren in Richtung des frontseitigen Endes der Sonde zu führen, und von dem Objekt zurückkommendes Objektlicht zum rückseitigen Ende der Sonde zu führen. Das Beleuchtungslicht kann insbesondere von einer Lichtquelle in der Steuervorrichtung der Sonde erzeugt werden, mittels einer Faserverbindung zur Sonde geführt und am rückseitigen Ende der Sonde, beispielsweise mittels einer Faserkupplung, in die erste Lichtleitfaser eingekoppelt werden. In umgekehrter Richtung kann das Objektlicht zur Auswertung zur Steuervorrichtung geführt werden.

Weiterhin umfasst die Sonde vorteilhaft eine Scanvorrichtung, die dazu eingerichtet ist, die erste Lichtleitfaser oder das aus der ersten Lichtleitfaser austretende Beleuchtungslicht zum Abtasten des Objekts bei dem scannenden bildgebenden Verfahren lateral abzulenken. Die Scanvorrichtung ist vorteilhaft ein bewegliches elektronisch ansteuerbares Element, insbesondere ein Faserscanner oder ein Spiegelscanner. Im Fall des Faserscanners erfolgt die Strahlablenkung vor dem Austritt des Beleuchtungslichts aus der ersten Lichtleitfaser durch Auslenkung der Faser. Im Fall des Spiegelscanners erfolgt die Strahlablenkung nach dem Austritt des Beleuchtungslichts aus der ersten Lichtleitfaser durch die Auslenkung eines Spiegels, auf den das Beleuchtungslicht trifft. Die Scanvorrichtung ermöglicht insbesondere eine zweidimensionale laterale Strahlablenkung, um das Objekt bei dem bildgebenden Verfahren in einem zweidimensionalen Raster abzutasten. In dem ersten Strahlengang sind somit zum einen eine Strahlführung und zum anderen eine aktive, elektronisch ansteuerbare Strahlablenkung für das bildgebende Verfahren realisiert.

Gemäß der Erfindung weist der zweite Strahlengang in der Sonde eine zweite Lichtleitfaser auf. Die zweite Lichtleitfaser ist vorteilhaft dazu eingerichtet, ein Anregungslicht oder ein detektiertes Objektlicht für das spektroskopische Verfahren zu führen. Die zweite Lichtleitfaser ist vorzugsweise parallel zur ersten Lichtleitfaser in der Sonde angeordnet. Die zweite Lichtleitfaser weist vorzugsweise einen Kerndurchmesser von 3 µm bis 300 µm und eine numerische Apertur von 0,08 bis 0,3 auf. Das Anregungslicht für das spektroskopische Verfahren kann insbesondere von einer Lichtquelle in der Steuervorrichtung der Sonde erzeugt werden, mittels einer Faserverbindung zur Sonde geführt und am rückseitigen Ende der Sonde, beispielsweise mittels einer Faserkupplung, in die zweite Lichtleitfaser eingekoppelt werden. In umgekehrter Richtung kann das Objektlicht zur Auswertung zur Steuervorrichtung geführt werden.

Gemäß der Erfindung weist die Sonde einen Strahlteiler-Filter auf. Der Strahlteiler-Filter ist dazu eingerichtet den ersten Strahlengang und den zweiten Strahlengang innerhalb der Sonde zumindest bereichsweise zusammenzuführen. Gemäß der Erfindung werden mittels des Strahlteiler-Filters der Strahlengang des scannenden bildgebenden Verfahrens und des spektroskopischen Verfahrens zumindest teilweise vereint. Die zumindest teilweise Vereinigung der Strahlengänge ist vorteilhaft, um in der miniaturisierten Sonde mit einem Durchmesser von insbesondere nicht mehr als 5 mm die Strahlengänge für beide Verfahren führen zu können. Insbesondere ermöglicht die Vereinigung der Strahlengänge die zumindest teilweise gleichzeitige Nutzung einer optischen Linsenanordnung in beiden Strahlengängen. Der Strahlteiler-Filter ist vorzugsweise als Strahlteilerplatte oder als Prismenstrahlteilerwürfel ausgeführt. Der Strahlteiler-Filter, beispielsweise ein Strahlteilerwürfel, ist vorteilhaft wellenlängenselektiv, insbesondere farbteilend.

Gemäß zumindest einer Ausführungsform ist die zweite Lichtleitfaser dazu eingerichtet, das Anregungslicht des spektroskopischen Verfahrens zu führen, und die erste Lichtleitfaser dazu eingerichtet, das detektierte Licht des spektroskopischen Verfahrens zu führen. Alternativ ist die erste Lichtleitfaser dazu eingerichtet, das Anregungslicht des spektroskopischen Verfahrens zu führen, und die zweite Lichtleitfaser dazu eingerichtet, das detektierte Licht des spektroskopischen Verfahrens zu führen. In beiden Fällen werden das Anregungslicht und das detektierte Licht des spektroskopischen Verfahrens vorteilhaft nicht in der gleichen Lichtleitfaser geführt. Auf diese Weise wird vorteilhaft vermieden, dass ein Störsignal, das durch das Anregungslicht des spektroskopischen Verfahrens in der ersten oder zweiten Lichtleitfaser entstehen könnte, dem detektierten Licht überlagert und zur Auswerteeinheit geführt wird.

Gemäß zumindest einer Ausführungsform ist der Strahlteiler-Filter wellenlängenselektiv. Der Strahlteiler-Filter ist insbesondere dazu eingerichtet, das Anregungslicht und das detektierte Licht des spektroskopischen Verfahrens voneinander zu trennen. Das detektierte Licht des spektroskopischen Verfahrens, d.h. das vom Objekt kommende Licht, weist insbesondere größere Wellenlängen als das Anregungslicht auf. Die Reflexion und die Transmission des Strahlteiler-Filters sind vorzugsweise derart eingestellt, dass das Anregungslicht des spektroskopischen Verfahrens im Wesentlichen transmittiert und nicht reflektiert wird, oder umgekehrt das Anregungslicht des spektroskopischen Verfahrens im Wesentlichen reflektiert und nicht transmittiert wird. Beispielsweise kann das Anregungslicht des spektroskopischen Verfahrens von dem Strahlteiler-Filter reflektiert und das detektierte Licht transmittiert werden. Alternativ kann das Anregungslicht des spektroskopischen Verfahrens von dem Strahlteiler-Filter transmittiert und das detektierte Licht reflektiert werden.

Gemäß zumindest einer Ausführungsform ist der Strahlteiler-Filter für das Beleuchtungslicht und für das vom Objekt zurückkommende Objektlicht des scannenden bildgebenden Verfahrens transmittierend. Anders als für das spektroskopische Verfahren ist der Strahlteiler-Filter für das bildgebende Verfahren in beiden Richtungen transmittierend. Insbesondere werden sowohl das Beleuchtungslicht als auch das Objektlicht des bildgebenden Verfahrens in der ersten Lichtleitfaser geführt.

Der erste optische Strahlengang und der zweite optische Strahlengang überlappen in der Sonde vorteilhaft zumindest teilweise. Insbesondere können der erste optische Strahlengang und der zweite optische Strahlengang am frontseitigen Ende der Sonde überlappen, wobei zumindest ein Linsenelement gleichzeitig in dem ersten optischen Strahlengang und dem zweiten optischen Strahlengang angeordnet sein kann. Dies ermöglicht vorteilhaft einen kompakten Aufbau der optischen Sonde mit wenigen optischen Komponenten.

Gemäß der Erfindung weist die optische Sonde einen Durchmesser von nicht mehr als 5 mm, insbesondere im Bereich zwischen 1 mm und 5 mm auf. Die Sonde ist somit eine miniaturisierte optische Sonde. Insbesondere kann es sich bei der optischen Sonde um eine endoskopische Sonde handeln. Durch die Miniaturisierung ermöglicht die Sonde vorteilhaft die Untersuchung des Objekts mit mindestens einem bildgebenden Verfahren und mindestens einem spektroskopischen Verfahren auf minimal-invasive Weise, insbesondere mittels Endoskopie.

Die Sonde umfasst gemäß zumindest einer Ausführungsform mindestens ein Linsenelement zur Strahlformung des Beleuchtungslichts und/oder des Objektlichts, wobei das mindestens eine Linsenelement zumindest bereichsweise zwischen der ersten Lichtleitfaser und dem frontseitigen Ende der Sonde angeordnet ist.

Bei einer Ausgestaltung der optischen Sonde ist die Scanvorrichtung ein Faserscanner, der insbesondere auf die erste Lichtleitfaser in dem ersten optischen Strahlengang einwirkt. Der Faserscanner ist vorzugsweise dazu eingerichtet, eine radiale Auslenkung der ersten Lichtleitfaser um nicht mehr als ± 1,2 mm zu bewirken. Der Faserscanner kann insbesondere ein piezoelektrischer Faserscanner sein. Der Faserscanner ist vorteilhaft von einer Steuervorrichtung der Sonde elektrisch ansteuerbar.

Bei einer alternativen Ausgestaltung der optischen Sonde ist die Scanvorrichtung ein Spiegelscanner. Der Spiegelscanner umfasst vorzugsweise einen Spiegel mit einem Durchmesser von nicht mehr als 2 mm. Vorzugsweise ist der Spiegelscanner dazu eingerichtet, eine Strahlauslenkung von nicht mehr als ± 20° zu bewirken. Der Spiegelscanner ist vorteilhaft von einer Steuervorrichtung der Sonde elektrisch ansteuerbar.

Der Spiegelscanner kann insbesondere ein MEMS-Mikrospiegelscanner sein.

Gemäß zumindest einer Ausgestaltung weist die erste Lichtleitfaser einen inneren Faserkern und einen äußeren Faserkern auf. Der innere Faserkern sollte vorzugsweise derart dimensioniert sein, dass er bei der Anregungswellenlänge des Verfahrens eine Einmodenfaser (single mode fiber) bildet. Der äußere Faserkern weist vorzugsweise einen Kerndurchmesser zwischen 20 µm und 300 µm und eine numerische Apertur zwischen 0,15 und 0,4 auf.

Gemäß zumindest einer Ausführungsform umfasst die optische Sonde mindestens ein Linsenelement, das eine Gradientenindexlinse aufweist oder aus einer Gradientenindexlinse besteht. Eine Gradientenindexlinse weist ein Brechzahlprofil auf, das vorteilhaft zur gezielten Korrektur optischer Abbildungsfehler eingestellt werden kann. Die Gradientenindexlinse ermöglicht eine solche Korrektur optischer Abbildungsfehler bei einer im Vergleich zu komplexen herkömmlichen Linsensystemen geringen Baugröße. Die Verwendung mindestens einer Gradientenindexlinse ist somit vorteilhaft, um einen besonders kompakten Aufbau der optischen Sonde zu erzielen.

Gemäß zumindest einer Ausführungsform enthält der zweite Strahlengang zumindest ein optisches Filter zur Selektion eines Wellenlängenbereichs für das spektroskopische Verfahren. Bei dem spektroskopischen Verfahren handelt es sich vorzugsweise um Raman-Spektroskopie. Bei der Raman-Spektroskopie können sich das Anregungslicht und das detektierte Licht im Objektraum überlagern. Insbesondere kann es sich um konfokale Raman-Spektroskopie handeln.

Bei einem Verfahren zum Betrieb der optischen Sonde wird ein Objekt, insbesondere zu untersuchende Objekt im Bereich der Biologie oder der Medizin, mittels der optischen Sonde mit mindestens einem scannenden bildgebenden Verfahren und mit mindestens einem spektroskopischen Verfahren untersucht.

Das scannende bildgebende Verfahren ist vorteilhaft eines der folgenden Verfahren: optische Kohärenztomografie (OCT), CARS-Mikroskopie, SRS-Mikroskopie, Multiphotonenmikroskopie, SHG-Multiphotonenmikroskopie, konfokale Einzelphotonen-Fluoreszenz, Reflexionsmikroskopie. Weiterhin ist das spektroskopische Verfahren vorzugsweise konfokale Raman-Spektroskopie. Die genannten Verfahren unterscheiden sich in ihrer Spezifität und Sensitivität. Für Anwendungen in der Medizin und der Biologie ist deshalb die Synergie von mindestens zwei solcher Verfahren von besonderem Vorteil.

Weitere vorteilhafte Ausgestaltungen des Verfahrens zum Betrieb der optischen Sonde ergeben sich aus der Beschreibung der optischen Sonde und umgekehrt.

Die Erfindung wird im Folgenden anhand von Ausführungsbeispielen im Zusammenhang mit den Figuren 1 bis 9 näher erläutert.

Es zeigen:
- Figur 1: eine schematische Darstellung einer optischen Anordnung zur Untersuchung eines Objekts mittels einer optischen Sonde gemäß einem Ausführungsbeispiel, und
- Figuren 2 bis 9: jeweils eine schematische Darstellung eines Querschnitts durch eine optische Sonde gemäß einem Ausführungsbeispiel.

Gleiche oder gleich wirkende Bestandteile sind in den Figuren jeweils mit den gleichen Bezugszeichen versehen. Die dargestellten Bestandteile sowie die Größenverhältnisse der Bestandteile untereinander sind nicht als maßstabsgerecht anzusehen.

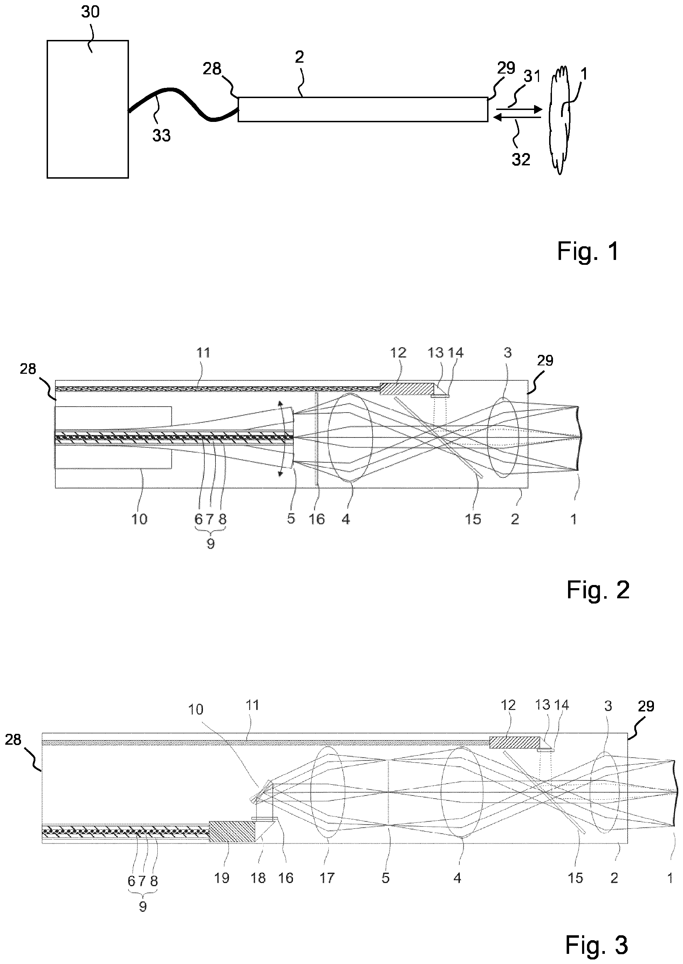

Figur 1 zeigt schematisch eine optische Anordnung zur Untersuchung eines Objekts 1 mittels einer optischen Sonde 2. Die optische Sonde 2 ist eine miniaturisierte bildgebende Sonde, die vorteilhaft für den endoskopischen Einsatz geeignet ist. Hierzu weist die optische Sonde vorteilhaft einen Durchmesser von nicht mehr als 5 mm, vorzugsweise im Bereich von 1 mm bis 5 mm auf. Das Objekt 1 kann insbesondere menschliches oder tierisches Gewebe sein.

Die optische Sonde 2 weist ein rückseitiges Ende 28 und ein frontseitiges Ende 29 auf. Das frontseitige Ende 29 der Sonde ist beim Betrieb der Sonde 2 dem zu untersuchenden Objekt 1 zugewandt. Am frontseitigen Ende 28 kann Beleuchtungslicht 31 aus der Sonde 2 ausgekoppelt und auf das Objekt 1 gerichtet werden, und vom Objekt 1 zurückkommendes Objektlicht 32 in die optische Sonde einkoppelt werden. Ein Objektfelddurchmesser der Sonde 2 beträgt vorzugsweise etwa 0,2 mm bis 3 mm, beispielsweise 0,2 mm bis 1 mm bei einer numerischen Apertur (NA) von 0,8 bis 0,15 und etwa 1 mm bis 3 mm bei einer NA von 0,15 bis 0,05.

Das rückseitige Ende 28 ist vorzugsweise mittels einer Verbindungsleitung 33, die eine oder mehrere Lichtleitfasern und elektrische Anschlussleitungen umfassen kann, an eine Steuervorrichtung 30 anschließbar. Die Steuervorrichtung 30 dient insbesondere zur Ansteuerung der Sonde 2 und zur Signalauswertung.

Mittels der Verbindungsleitung 33 kann Beleuchtungslicht 31 von der Steuervorrichtung 30 zur Sonde und Objektlicht 32 von der Sonde 2 zur Steuervorrichtung 30 geführt werden. Die Steuervorrichtung 30 kann insbesondere eine oder mehrere Lichtquellen zum Betrieb der optischen Sonde 2 aufweisen, die vorteilhaft mittels Lichtleitfasern in der Verbindungsleitung 33 mit der Sonde 2 verbunden sind.

Die Steuervorrichtung 30 kann weiterhin Komponenten zur optischen, insbesondere spektroskopischen Analyse, des von der Sonde empfangenen Objektlichts 32 aufweisen. Die Steuervorrichtung 30 kann außerdem Komponenten zur elektronischen Bildverarbeitung und -darstellung aufweisen oder mit dazu geeigneten Geräten verbunden sein.

Die optische Sonde 2 ist vorteilhaft zur Untersuchung des Objekts 1, insbesondere von Gewebe, mit mindestens zwei verschiedenen optischen Verfahren geeignet. Zu diesem Zweck geeignete Ausgestaltungen der optischen Sonde 2 werden im Zusammenhang mit den folgenden Ausführungsbeispielen beschrieben.

Figur 2 zeigt ein erstes Ausführungsbeispiel der optischen Sonde 2, die zur Untersuchung eines Objekts 1, insbesondere von Gewebe, mit mindestens zwei optischen Verfahren geeignet ist. Das eine Verfahren ist vorteilhaft ein quasi-konfokal bildgebendes Verfahren, bei dem Beleuchtungslicht in einer ersten Lichtleitfaser 9 an das frontseitige Ende der optischen Sonde 2 geleitet wird. Das aus einem inneren Faserkern 6 der ersten Lichtleitfaser 9 austretende Beleuchtungslicht durchläuft einen ersten für die Beleuchtungswellenlänge spektral durchlässigen Filter 16, wird mit einem ersten Linsenelement 4 gebündelt, durchläuft einen weiteren für die Beleuchtungswellenlänge spektral durchlässigen geneigten Strahlteiler-Filter 15 und wird mit einem weiteren Linsenelement 3 auf das zu untersuchende Objekt 1 fokussiert. Das erste Linsenelement 3 und das zweite Linsenelement 4 können jeweils eine Linse oder eine Linsengruppe umfassen.

Das Beleuchtungslicht wird je nach Beschaffenheit des Objekts 1, insbesondere der Gewebemorphologie oder -zusammensetzung, absorbiert, gestreut oder reflektiert. Das dabei vom Objekt 1 ausgehende Objektlicht wird durch die optische Anordnung umfassend die Linsenelemente 3, 4 und die Filter 15, 16 wieder aufgesammelt, transmittiert und konfokal in die erste Lichtleitfaser 9 abgebildet bzw. eingekoppelt. Die erste Lichtleitfaser 9 weist einen zweiten konzentrisch um den inneren Faserkern 6 angeordneten äußeren Faserkern 7 auf, der durch einen Mantel 8 geschützt ist. Licht vom Objekt 1 kann sowohl in den inneren Faserkern 6, durch den das Beleuchtungslicht geführt wird, als auch in den größeren äußeren Faserkern 7 eingekoppelt werden. Dies beruht darauf, dass eine totale Konfokalität in der Regel nicht vollständig gegeben ist, weil das Licht vom Objekt auch aus tieferen oder flacheren Gewebeschichten rückgestreut wird, bzw. im Fall einer Fluoreszenz im Objekt auch spektral zum Beleuchtungslicht verschoben sein kann.

Am rückseitigen Ende 28 der Sonde 2 kann eine getrennte Ein- und Auskopplung des Lichtes in den inneren Faserkern 6 und äußeren Faserkern 7 durch geeignete Faserkoppler (nicht dargestellt) durchgeführt werden. Der innere Faserkern 6 kann insbesondere als single-mode-Faser fungieren, beispielsweise im Wellenlängenbereich von etwa 800 nm bis 1600 nm, wenn die Sonde 2 für die Optische Kohärenztomographie (OCT) vorgesehen ist, oder beispielsweise im Wellenlängenbereich zwischen 400 und 1500 nm, wenn die Sonde für Multiphotonen-, CARS- , Fluoreszenz- oder SRS-Mikroskopie vorgesehen ist. Der äußere Faserkern 7 fungiert insbesondere als Multimode-Faser und weist beispielsweise einen Kerndurchmesser zwischen 20 und 300 µm und einer numerische Apertur zwischen 0,15 und 0,4 auf.

Die Bildgebung beruht bei dem Ausführungsbeispiel gemäß Figur 2 darauf, dass die erste Lichtleitfaser 9 durch einen Faserscanner 10 lateral ausgelenkt werden kann. Der Faserscanner 10 ermöglicht vorteilhaft eine radiale Auslenkung der Faserspitze von vorzugsweise etwa 1 mm im Durchmesser, beispielsweise bis zu etwa 1,2 mm. Durch laterales Auslenken der Faserspitze durch den Faserscanner 10 in der damit aufgespannten Zwischenbildfläche 5 wird das Objekt 1 fokussiert abgerastert.

Als bildgebende Verfahren eignen sich insbesondere Folgende:
- Anregung und Detektion bei gleicher Wellenlänge, wobei das in den inneren Faserkern 6 rückgestreute Objektlicht zur Bildgebung dient. Im Fall der optischen Kohärenztomographie wird dabei zusätzlich der optische Weg vom Austritt aus der ersten Lichtleitfaser 9 zum Objekt 1 und zurück in die erste Lichtleitfaser 9 durch eine interferometrische Auswertung extern bestimmt, so dass damit eine 3D-Rekonstruktion der Struktur des Objekts 1 möglich ist.
- Anregung einer Einphotonenfluoreszenz im zu untersuchenden Objekt 1 durch die Beleuchtungswellenlänge. Das langwellig verschobene, aus dem Objekt 1 emittierte Licht passiert das Linsenelement 3, den Filter 15, das Linsenelement 4 und den Filter 16 und wird in den äußeren Faserkern 7 eingekoppelt und als Funktion der Faserauslenkung detektiert. Der äußere Faserkern 7, der typischerweise einen größeren Kernquerschnitt und eine höhere numerische Apertur als der innere Faserkern 6 hat, dient dabei als konfokale Blende. Die Konfokalität kann durch die geeignete Abstimmung von Kerndurchmesser und NA gezielt beeinflusst werden.
- Anregung einer Zwei- oder Dreiphotonenfluoreszenz bzw. Erzeugung der zweiten Harmonischen (SHG) im zu untersuchenden Objekt 1 durch die Beleuchtungswellenlänge. Dabei handelt es sich um einen nicht-linearen Absorptionsprozess bei hohen Anregungsleistungen oder Intensitäten. Das zu detektierende SHG-Licht bzw. Fluoreszenzlicht entsteht bei oder oberhalb der halben Wellenlänge (2-Photonenfluoreszenz) bzw. eines Drittels der Anregungswellenlänge, jedoch deutlich unterhalb der Anregungswellenlänge selbst. Auch in diesen Wellenlängenbereichen sind die Filter 15, 16 vorteilhaft in Transmission durchlässig. Das vom Objekt 1 emittierte Licht aus dem Umgebungsbereich des Anregungsspots wird über die Linsenelemente 3, 4 sowie durch die Filter 15, 16 in quasikonfokaler Weise auf die scannende erste Lichtleitfaser 9 geleitet und in den äußeren Faserkern 7 eingekoppelt. Der äußere Faserkern 7 bildet damit eine konfokale Blende, die durch den Durchmesser des äußeren Faserkerns 7 und dessen numerische Apertur (NA) bestimmt wird. Je nachdem, wie groß der äußere Faserkern 7 und die NA gewählt werden, kann die Konfokalität abgeschwächt und die Sammeleffizienz erhöht werden. Die Konfokalität ergibt sich in diesem Fall schon dadurch, dass das SHG-, 2- oder 3-Photonenfluoreszentlicht aufgrund der Nichtlinearität des Anregungsprozesses sowieso nur im Fokusvolumen des Anregungsspots entsteht, wo die Anregungsintensität entsprechend hoch ist.
- Anregung eines Kohärenten Anti-Stokes-Raman-Streusignals (CARS) bzw. eines Stimulierten Raman-Streusignals im Objekt 1. Hierbei handelt es sich um einen nicht-linearen optischen Prozess, bei dem zueinander kohärente Laserpulse verschiedener Wellenlängen im inneren Faserkern 6 in Richtung des frontseitigen Endes 29 der Sonde 2 geleitet und über die bei diesen Wellenlängen transmittierenden Filter 16, 15 und die Linsenelemente 4, 3 auf das Objekt 1 fokussiert werden. In beiden Fällen werden in dem Objekt 1 bestimmte Molekülschwingungen angeregt, deren emittierte optische Strahlung bei kürzeren Wellenlängen ein quantitatives Signal über die Konzentration des Moleküls in dem Objekt 1 darstellt. Das Objektlicht wird im Fall von CARS durch die Linsenelemente 3, 4 sowie durch die bei diesen CARS-Wellenlängen durchlässigen Filter 15, 16 in den äußeren Faserkern 7 eingekoppelt, so dass es zur Bildgebung als Funktion der Faserauslenkung und damit der lateralen Objektebenenposition detektiert werden kann. Für die beschriebenen bildgebenden Verfahren wird mit den Linsenelementen 3, 4 und Filtern 15, 16 vorteilhaft eine hohe Abbildungsqualität der optischen Anordnung erzielt.

Bei der optischen Sonde 2 wird das scannend bildgebende Verfahren vorteilhaft mit einer zweiten Ramanspektroskopischen Modalität in stark miniaturisierter Weise gekoppelt. Da das Raman-Signal sehr schwach ist, eine hochauflösende Bildgebung durch lange Integrationszeiten deshalb für klinische oder in vivo-Untersuchungen nicht sinnvoll erscheint, und eine hoch wirksame spektrale Trennung zwischen Anregungs- und Raman-Signal notwendig ist, erfolgt bei der optischen Sonde 2 vorteilhaft eine punktuelle konfokale Raman-Anregung und -Detektion im Zentrum des Bildfeldes.

In einer ersten Ausführungsform wird das Raman-Anregungslicht, typischerweise ein Laserlicht, bei einer insbesondere stabilisierten Wellenlänge (z. B. 785 nm), über eine parallel zur ersten Lichtleitfaser 9 angeordnete zweite Lichtleitfaser 11 an das frontseitige Ende 29 der Sonde geführt. Die zweite Lichtleitfaser 11 weist vorzugsweise einen Kerndurchmesser von etwa 3 - 300 µm auf. Die NA der zweiten Lichtleitfaser 11 beträgt vorzugsweise etwa 0,08 bis 0,4. Das Raman-Anregungslicht wird vorzugsweise über ein Strahlformungselement 12 in seiner Divergenz angepasst und über ein Umlenkelement 13 auf den geneigten, Strahlteiler-Filter 15 geleitet. Ein an dem Strahlformungselement 12 bzw. am Umlenkelement 13 angebrachter spektraler Filter 14 lässt nur das schmalbandige Raman-Anregungslicht durch und filtert vor allem den in der zweiten Lichtleitfaser 11 induzierten intrinsischen Raman-Untergrund heraus (sogenannter Laser Cleaning Filter), der in dem Spektralbereich liegen würde, der letztendlich von dem Objekt 1 als Raman-Signal emittiert wird. Der Strahlteiler-Filter 15 ist für die Wellenlänge der Raman-Anregung vorteilhaft hoch reflektierend, so dass das Licht zum Objekt 1 geleitet wird. Das Umlenkelement 13 und der Strahlteiler-Filter 15 sind so angeordnet, dass das Raman-Anregungslicht einen Beleuchtungsfleck auf dem Objekt 1 bildet, der nahezu konzentrisch zur optischen Achse (on-axis) des scannenden Systems ist (gestrichelte Linie in Figur 2). Das Raman-Licht, welches in einem von der Raman-Anregung beleuchteten Spotvolumen entsteht und spektral zu größeren Wellenlängen bezüglich der Raman-Anregung verschoben ist, wird zu einem möglichst großen Anteil durch das Linsenelement 3 aufgesammelt und gebündelt. Die spektralen Filter 15 und 16 sind so ausgelegt, dass sie das Raman-Detektionslicht möglichst effizient transmittieren. Das Linsenelement 4 fokussiert das gebündelte Raman-Signal auf die inneren und äußeren Faserkerne 6, 7 der ersten Lichtleitfaser 9. Die erste Lichtleitfaser 9 leitet das Raman-Signal an das rückseitige Ende der Sonde 2, wo es über einen geeigneten Faserkoppler oder -schalter in ein Spektrometer gekoppelt werden kann, welches das Raman-Spektrum analysiert. Der Filter 16 hat eine umgekehrte Funktion zum Filter 14. Er blockiert den spektralen Anteil der Raman-Anregung, so dass das zu detektierende Raman-Spektrum nicht von einem starken Anregungspeak überlagert wird. Das Raman-Signal wird aufgrund der Konfokalität der Anregung und -Detektion dann am größten sein, wenn die erste Lichtleitfaser 9 durch den Faserscanner 10 nicht ausgelenkt wird, sondern sich auf der optischen Achse befindet. Allerdings können Montagetoleranzen des optischen Systems gegebenenfalls durch eine definierte statische bzw. auch dynamische Auslenkung der ersten Lichtleitfaser 9 durch den Faserscanner 10 kompensiert werden.

### Beispiel 1:

Die Raman-Anregung findet bei einer Wellenlänge von etwa 785 nm statt. In diesem Fall dient der spektrale Filter 14 als Bandpass von 780 bis 790 nm oder als Tiefpass für Wellenlängen unterhalb 790 nm und weist zwischen 800 und 1000 nm eine starke Sperrwirkung auf. Die Filter 15, 16 weisen vorzugsweise eine hohe Reflexion von 780 nm - 790 nm sowie eine hohe Transmission im Spektralbereich von 400 nm bis 775 nm und von 800 nm - 1400 nm auf. Dadurch wird das Raman-Spektrum des Objekts 1 zwischen 800 nm und 1000 nm auf die erste Lichtleitfaser 9 geleitet.

Im Fall einer Einphotonenfluoreszenz-Bildgebung kann Anregungslicht im Bereich von 400 nm - 550 nm aus dem inneren Faserkern 6 auf das Objekt 1 fokussiert werden. Das Fluoreszenzlicht oberhalb der Anregungswellenlänge, aber unterhalb von 775 nm wird durch die inneren und äußeren Faserkerne 6, 7 detektiert und kann durch Farbteiler vom Anregungslicht separiert werden. Im Fall einer OCT-Bildgebung kann Licht im Bereich von 800 nm - 1400 nm vom inneren Faserkern 6 sowohl auf das Objekt 1 gesendet als auch das rückgestreute oder reflektierte Licht des Objekts 1 durch den inneren Faserkern 6 empfangen werden.

Im Fall einer Multiphotonenmikroskopie-Bildgebung wird Anregungslicht im Bereich unter 780 nm bzw. oberhalb von 800 nm vom inneren Faserkern 6 auf das Objekt 1 fokussiert. Das SHG- bzw. Multiphotonenfluoreszenzlicht zwischen 400 und 775 nm wird durch den äußeren Faserkern 7 empfangen. Im Fall einer CARS- oder SRS-Bildgebung wird Licht der Pump- und Stokes-Anregung im Spektralbereich zwischen 800 nm und 1400 nm vom Faserkern 6 auf das Objekt geleitet. Das CARS-Signal unterhalb von 775 nm kann durch den äußeren Faserkern 7 effizient empfangen werden.

In einer alternativen zweiten Ausführungsform kann die Raman-Anregung auch durch den inneren Faserkern 6 erfolgen, wobei dann die Filter 15, 16 sowohl für die Raman-Anregung als auch für die Wellenlängen des bildgebenden Verfahrens transmittierend sind, jedoch eine starke Sperrwirkung in dem Spektralbereich oberhalb der Raman-Anregungswellenlänge aufweisen, wo Raman-Spektren detektiert werden sollen. Diese Sperrwirkung ist vorteilhaft, um faserintrinsische Eigenfluoreszenz in diesem Spektralbereich auszufiltern und nicht auf das Objekt 1 zu fokussieren. Der geneigte Strahlteiler-Filter 15 weist eine hohe Reflexion im Bereich des zu detektierenden Raman-Spektrums auf. Die punkt- oder messfleckförmige Detektion des Raman-Lichtes vom Objekt 1 erfolgt in diesem Fall durch die konfokal zur ersten Lichtleitfaser 9 angeordnete faseroptische Raman-Detektionseinheit umfassend den Filter 14, welcher eine hohe Transmission im Raman-Detektionsbereich, jedoch eine starke Sperrwirkung im Raman-Anregungsbereich besitzt, die Umlenkeinheit 13, das Strahlformungselement 12 und die zweite Lichtleitfaser 11, die als Raman-Kollektionsfaser fungiert.

### Beispiel 2:

Bei einem Ausführungsbeispiel findet die Raman-Anregung bei einer Wellenlänge von etwa 785 nm statt. Die Filter 15, 16 weisen vorteilhaft zwischen 400 nm und 795 nm eine hohe Transmission, zwischen 800 nm und 1000 nm eine sehr starke Sperrwirkung und zwischen 1000 nm und 1300 nm wieder eine hohe Transmission auf. Der Strahlteiler-Filter 15 hat im Bereich zwischen 800 nm und 1000 nm eine hohe Reflexion. Dadurch kann das Raman-Spektrum des Objekts 1 zwischen etwa 800 nm und 1000 nm mittels des Umlenkelements 13 und der zweiten Lichtleitfaser 11 in Richtung des rückseitigen Endes 28 der Sonde 2, insbesondere zur Detektion in einer angeschlossenen Auswerteeinheit, geleitet werden. Im Fall einer Einphotonenfluoreszenz-Bildgebung kann Anregungslicht im Wellenlängenbereich von beispielsweise 400 nm - 550 nm aus dem inneren Faserkern 6 auf das Objekt 1 fokussiert werden. Das Fluoreszenzlicht oberhalb der Anregungswellenlänge, aber unterhalb von 795 nm kann durch die inneren und äußeren Faserkerne 6, 7 empfangen und durch Farbteiler vom Anregungslicht separiert werden. Im Fall einer OCT-Bildgebung kann Licht im Bereich von 1000 nm - 1400 nm aus dem inneren Faserkern 6 auf das Objekt 1 gerichtet und auch das rückgestreute oder reflektierte Licht des Objekts 1 vom inneren Faserkern 6 empfangen werden. In diesen Fällen kann die erste Lichtleitfaser 9 auch als Einkernfaser (mit einem Faserkern 6) ausgeführt sein. Im Fall einer Multiphotonenmikroskopie-Bildgebung kann Anregungslicht im Bereich unter 780 nm bzw. insbesondere im Fall einer 3-Photonenfluoreszenz oberhalb von 1000 nm aus dem inneren Faserkern 6 auf das Objekt 1 fokussiert werden. Das Multiphotonenfluoreszenzlicht zwischen 400 nm und 795 nm kann durch den äußeren Faserkern 7 aufgesammelt werden. Im Fall einer CARS- oder SRS-Bildgebung kann Licht der Pump- und Stokes-Anregung im Spektralbereich unterhalb von 795 nm und zwischen 1000 nm und 1400 nm aus dem inneren Faserkern 6 auf das Objekt 1 geleitet werden. Das CARS-Signal unterhalb von 795 nm kann durch den äußeren Faserkern 7 empfangen werden.

In Figur 3 ist ein weiteres Ausführungsbeispiel der optischen Sonde 2 gemäß einer dritten Ausführungsform dargestellt. Bei dieser Ausführungsform basiert die Bildgebung auf einem Spiegelscanner 10. Das Beleuchtungslicht der Bildgebung wird im inneren Faserkern 6 der parallel zur Achse der Sonde 2 angeordneten ersten Lichtleitfaser 9 zum frontseitigen Ende 29 der Sonde 2 geführt. Das aus der ersten Lichtleitfaser 9 austretende Beleuchtungslicht wird vorteilhaft von einem strahlformenden optischen Element 19 kollimiert, fokussiert oder in seiner Divergenz reduziert. Ein Umlenkelement 18 leitet das Beleuchtungslicht auf den Spiegelscanner 10. Dieser Spiegelscanner 10 lenkt das Beleuchtungslicht durch Reflexion in einem Winkel bezüglich der optischen Achse der Sonde 2 aus. Ein dem Spiegelscanner 10 nachfolgendes optisches Element 17 erzeugt aus diesen abgelenkten, kollimierten bzw. schwach divergenten Bündeln eine Zwischenbildebene 5. Diese Zwischenbildebene 5 ersetzt damit die Faserscan-Ebene der Ausführungsformen nach Figur 2. Die Brennweiten, Positionen und Ablenkwinkel der optischen Komponenten 10, 17, 18, 19 werden dabei so gewählt, dass sich für alle Scanwinkel, die das Bildfeld der gesamten Sonde 2 aufspannen, eine minimale Abschattung und Vignettierung im Bereich zwischen der ersten Lichtleitfaser 9 und dem Objekt 1 ergibt. Die multimodale Bildgebung und Raman-Detektion erfolgt ansonsten analog zu den beiden ersten Ausführungsformen. Der Filter 16 kann dabei in den Zwischenräumen zwischen dem Ablenkelement 18 und dem Linsenelement Optik 4 angeordnet sein. Eine besonders günstige Position ergibt sich jedoch auf der Austrittsfläche vom Umlenkelement 18 bzw. im Zwischenraum zwischen dem Umlenkelement 18 und dem Spiegelscanner 10, da hier das Bündel sehr eng begrenzt ist und kleine Divergenzwinkel besitzt.

Für alle beschriebenen bildgebenden Verfahren wird eine hohe Abbildungsqualität der optischen Sonde 2 gefordert, damit zum einen eine hohe Auflösung, zum anderen aber auch hohe Photonenausbeuten bzw. Sammeleffizienzen bei der konfokalen Bildgebung erzielt werden. Die optische Sonde 2 wird dabei vorzugsweise so gestaltet, dass sie bei maximaler Kompaktheit des Systems, ein möglichst großes Objektfeld mit einer möglichst hohen numerischen Apertur abtastet und somit die Etendue maximiert. Darüber hinaus sind die sphärische Aberration und off-axis-Abbildungsfehler, wie Koma und Astigmatismus, vorteilhaft so gut korrigiert, dass das aus dem als single-mode-Anregungsfaser fungierenden inneren Faserkern 6 austretende Licht nahezu beugungsbegrenzt innerhalb des gesamten Objektfeldes fokussiert werden kann und keine oder kaum Vignettierungsverluste auftreten. Aufgrund der Konfokalität der Anordnung für alle Modalitäten ist es darüber hinaus notwendig, eine Korrektur der Farblängs- und Querfehler für die jeweils beteiligten Wellenlängen zu erreichen. Für die im Folgenden beschriebenen Kombinationen mit einem Raman-Spektroskopie-Verfahren betrifft dies die Korrektur für folgende Wellenlängen:
- OCT bzw. Reflexions- und Streulichtbildgebung: OCT-Wellenlängenbereich (typisch 1200 nm - 1400 nm) bzw. sichtbarer Spektralbereich zwischen 440 und 650 nm, sowie die Raman-Detektion bzw. -Anregung (je nach Ausführungsform)
- Einphotonenfluoreszenz: die Fluoreszenz-Anregung- und Detektion (typisch 400 nm - 700 nm) sowie die Raman-Detektion bzw. -Anregung (je nach Ausführungsform)
- Zwei - und Dreiphotonenfluoreszenz: Anregung (typisch zwischen 750 nm und 1300 nm), Fluoreszenz (typisch zwischen 400 und 600 nm), sowie die Raman-Detektion bzw. -Anregung (je nach Ausführungsform)
- CARS und SRS-Mikroskopie: sehr gute Korrektur für Pump- und Stokes-Wellenlänge (typisch 750 nm - 850 nm und 1000 bis 1200 nm), da es eine sehr gute Überlappung zwischen den Anregungsfoki für beide Wellenlängen in der Objektebene geben muss, darüber hinaus für die CARS-Detektion (typisch zwischen 550 und 750 nm), sowie die Raman-Detektion bzw. -Anregung (je nach Ausführungsform).

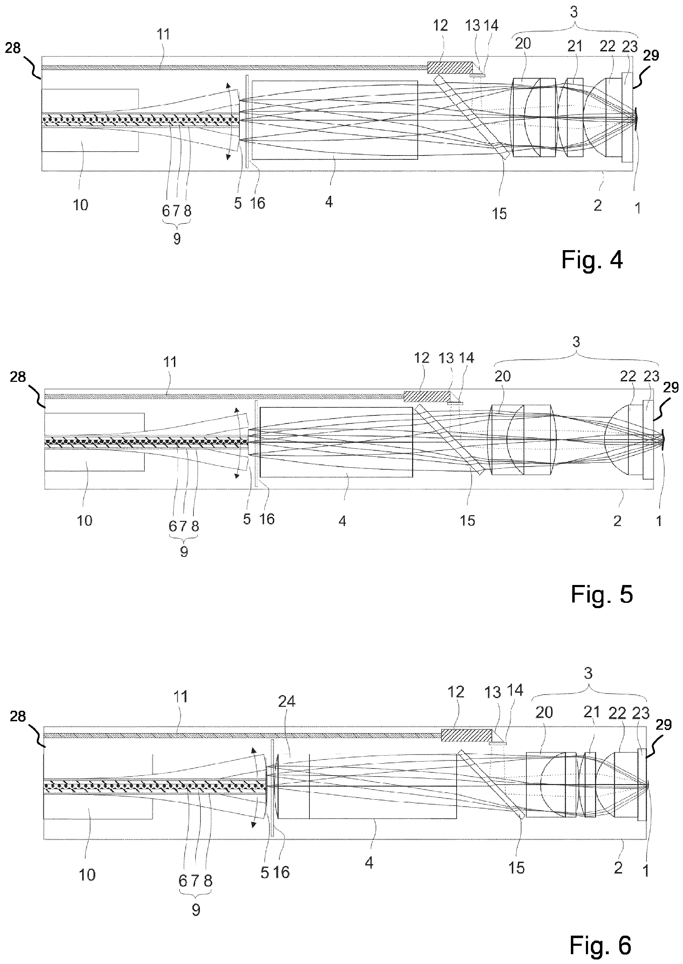

Diese Korrektur kann in Ausführungsformen der optischen Sonde 2 mit einem Faserscanner 10 (wie in Figur 2) insbesondere wie im Folgenden beschrieben erreicht werden. In Figur 4 ist ein Ausführungsbeispiel der optischen Sonde gezeigt, bei dem das Linsenelement 4 eine Gradientenindex-(GRIN)-Linse mit radialem Brechzahlprofil und senkrechten Stirnflächen ist. Der Strahlteiler-Filter 15 ist vorzugsweise als eine dünne Platte, die zu einem geringfügigen Strahlversatz in Transmissionsrichtung führt, oder als Prismenstrahlteilerwürfel ausgeführt. Das frontseitige Linsenelement 3 besteht bei dem Ausführungsbeispiel aus einer sphärischen Doublet-Linse 20, einer Plankonvex-Linse 21, einer weiteren Plankonvexlinse 22 und einem optionalen Frontfenster 23, welches vorteilhaft eine kratzfeste Oberfläche aufweist und eine hermetische Abdichtung der Sonde 2 bewirkt. Die Farbkorrektur wird durch eine abgestimmte Auswahl der Gläser der refraktiven Linsen mit der GRIN-Linse 4 erreicht. Die GRIN-Linse 4 ist in ihrem Brechzahlprofil so angepasst, dass die sphärische Aberration, sowie Astigmatismus und Koma des Gesamtsystems so korrigiert sind, dass sich eine nahezu beugungsbegrenzte Abbildungsqualität ergibt. Diese Ausführungsform ist insbesondere dann günstig, wenn eine hohe numerische Apertur im Bereich von 0,45 bis 1,0 im Objektraum erreicht werden soll, z. B. für die konfokale Einphotonen-Fluoreszenzmikroskopie, die Multiphotonenmikroskopie oder die CARS- bzw. SRS-Mikroskopie.

In Figur 5 ist ein weiteres Ausführungsbeispiel der optischen Sonde 2 dargestellt, bei dem im Unterschied zum Ausführungsbeispiel der Figur 4 die Plankonvex-Linse 21 in dem frontseitigen Linsenelement 3 nicht vorhanden ist. Auf die Plankonvex-Linse 21 kann insbesondere dann vorteilhaft verzichtet werden, wenn die numerische Apertur etwa 0,45 bis 0,6 beträgt.

In einer in Figur 6 dargestellten besonderen Ausführungsform, bei der die NA des inneren Faserkerns 6 der ersten Lichtleitfaser 9 beispielsweise größer als 0,18 und die laterale Auslenkung der Spitze der ersten Lichtleitfaser 9 beispielsweise mehr als 60% des Linsenradius der GRIN-Linse 4 beträgt, wird vorteilhaft eine weitere Plankonvexlinse 24 rückseitig auf die GRIN-Linse 4 aufgesetzt. Auf diese Weise ist es möglich, die Vignettierung stark zu reduzieren und insgesamt ein gut korrigiertes System zu erhalten.

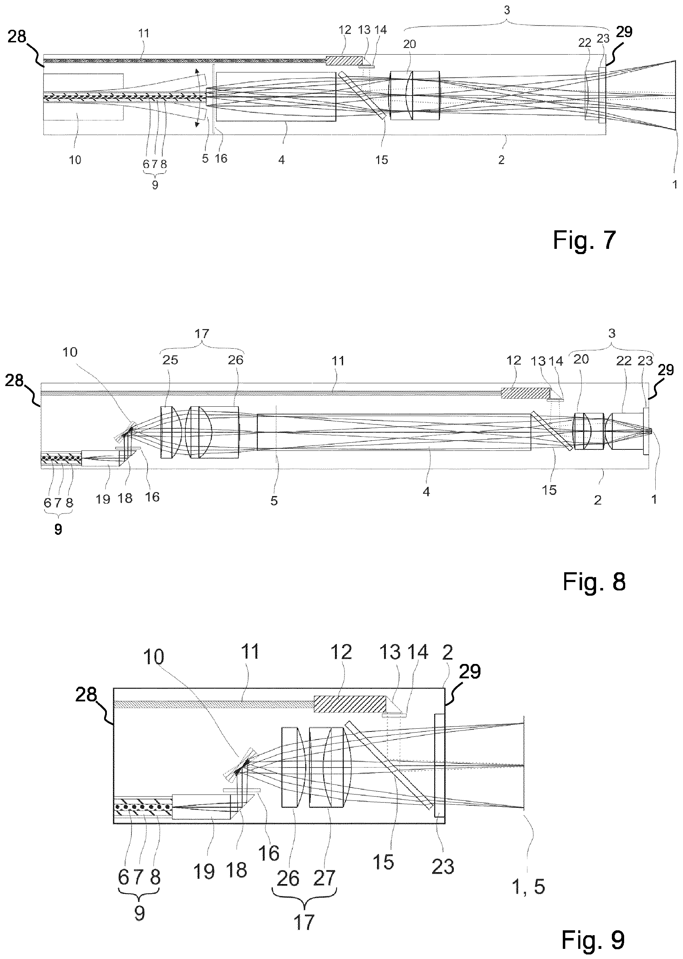

Figur 7 zeigt ein weiteres Ausführungsbeispiel der optischen Sonde 2. Im Fall der OCT und der klassischen endoskopischen Bildgebung können größere Objektfelder mit einer niedrigen numerischen Apertur abgescannt werden. Dazu hat es sich als besonders vorteilhaft erwiesen, für die Ausgestaltung mit dem Faserscanner 10 eine Anordnung nach Figur 7 zu verwenden. Das Linsenelement 4 dieser Anordnung ist vorzugsweise eine GRIN-Linse mit radialem Brechzahlprofil und ebenen senkrechten Stirnflächen. Das frontseitige Linsenelement 3 besteht bevorzugt aus einer Doublet-Linse 20, einer Plankonkav-Linse 22 und einem optionalen Frontfenster 23, welches insbesondere die Kratzfestigkeit und Dichtigkeit der Sonde 2 gewährleistet. Durch eine Plankonkav-Linse 22 wird vorteilhaft ein großes Bildfeld mit geringer NA erreicht. Aufgrund der vergleichsweise geringen Objekt-NA und einem großen Bildfeld kann bei diesem Ausführungsbeispiel auf die in einigen vorherigen Ausführungsbeispielen vorhandene Plankonvexlinse 21 verzichtet werden. Eine gute Farbkorrektur kann durch eine abgestimmte Auswahl der Gläser der refraktiven Linsen mit der GRIN-Linse 4 erreicht werden. Die GRIN-Linse 4 ist in ihrem Brechzahlprofil so angepasst, dass die sphärische Aberration, sowie Astigmatismus und Koma des Gesamtsystems so korrigiert sind, dass sich eine nahezu beugungsbegrenzte Abbildungsqualität ergibt. Diese Ausführungsform ist insbesondere dann günstig, wenn eine geringe numerische Apertur kleiner 0,1 im Objektraum erreicht werden soll, z. B. für OCT oder endoskopische Bildgebung mit großer Schärfentiefe.

In Figur 8 ist ein weiteres Ausführungsbeispiel der optischen Sonde dargestellt, bei dem die Bilderzeugung durch einen miniaturisierten Spiegelscanner 10 erfolgt. Bei diesem Ausführungsbeispiel ist das dem Spiegelscanner 10 nachfolgende optische Element 17 vorteilhaft durch ein System aus zwei refraktiven sphärischen Linsen realisiert, insbesondere aus einer Meniskuslinse 25 und einer Doublet-Linse 26. Diese erzeugen aus dem kollimierten oder nur schwach divergenten Bündel, welches nach dem strahlformenden optischen Element 19 und dem Umlenkelement 18 auf den Spiegelscanner 10 trifft, eine Zwischenbildebene 5. Dabei sind die Komponenten so aufeinander abgestimmt, dass diese Zwischenbildebene 5 bezüglich sphärischer Aberrationen, Astigmatismus, Koma und chromatischen Fehlern nahezu beugungsbegrenzt für das gesamte nutzbare Bildfeld korrigiert ist, welches der Spiegelscanner 10 aufspannt. Außerdem wird durch das optische Element 17 eine nahezu telezentrische Führung des Beleuchtungslichts in der Zwischenbildebene 5 gewährleistet, die eine Vignettierung des Beleuchtungslichts im weiteren Verlauf des Strahlengangs über das Linsenelement 4, den Strahlteiler-Filter 15 und das frontseitige Linsenelement 3 minimiert. Dadurch wird eine gleichmäßige Helligkeit im Bereich des Objekts 1 erreicht.

Für eine mikroskopische Bildgebung mit hoher numerischer Apertur und der damit verbundenen hohen Auflösung in der Objektebene ist vorzugsweise das erste Linsenelement 4 eine GRIN-Linse, der Strahlteiler-Filter 15 eine Strahlteilerplatte und das frontseitige Linsenelement 3 eine Linsenanordnung umfassend eine Doublet-Linse 20, eine Plankonvex-Linse 21, eine weitere Plankonvexlinse 22 und ein optionales Frontfenster 23. Das gesamte optische System ist vorteilhaft so ausgelegt, dass sich über das gesamte Objektfeld eine nahezu beugungsbegrenzte Abbildungsqualität für die relevanten Wellenlängen der Bildgebungsmodalität ohne markante Vignettierung ergibt.

Analog zur ersten und zweiten Ausführungsform kann die punktförmige Raman-Anregung oder Detektion in einem zweiten Strahlengang, der die zweite Lichtleitfaser 11, das Strahlformungselement 12, das Umlenkelement 13, den Filter 14 und den Strahlteiler-Filter 15 umfasst, erfolgen. Korrespondierend wird dann die Raman-Detektion oder Raman-Anregung im ersten Strahlengang umfassend den Filter 15, die Linsenelemente 4, 5, 25, 26, den Spiegelscanner 10, den Filter 16, das Umlenkelement 18 und das strahlformende optische Element 19 in bzw. durch die erste Lichtleitfaser 9 realisiert. In diesem Fall befindet sich der Spiegelscanner 10 in der neutralen Ruheposition, so dass die Konfokalität zwischen Anregung und Detektion gegeben ist.

Figur 9 zeigt ein weiteres Ausführungsbeispiel der optischen Sonde 2. Bei diesem Ausführungsbeispiel erfolgt eine scannende Bildgebung durch einen miniaturisierten Spiegelscanner 10 mit einem vergleichsweise großen Objektfeld und einer geringen numerischen Apertur zwischen 0,05 und 0,12, wie sie für die optische Kohärenztomographie (OCT) wünschenswert sind. Im Vergleich zu den vorherigen Ausführungsbeispielen wird mit einem vorteilhaft vereinfachten optischen System eine große Schärfentiefe der Abbildung im zu untersuchenden Objekt 1 (z. B. Gewebe) erzielt, und es kann eine nahezu beugungsbegrenzte Abbildungsqualität über das gesamte Bildfeld erreicht werden. Das dem Spiegelscanner 10 nachfolgende optische Element 17 besteht bei dieser Ausführungsform nur aus einer Plankonvex-Linse 26 und einer Doublet-Linse 27. Aufgrund des insgesamt nur sehr schwach divergenten Strahlengangs kann die Zwischenbildebene 5 gleichzeitig als Objektebene fungieren. Auf die bei den vorherigen Ausführungsbeispielen verwendeten Linsenelemente 3, 4 kann vorteilhaft verzichtet werden. Die Sonde 2 wird zum Schutz vorzugsweise nur noch durch ein Frontfenster 23 abgeschlossen. Die Raman-Anregung und - Detektion können analog zu den zuvor beschriebenen Beispielen erfolgen, wobei das Strahlformungselement 12 und das Umlenkelement 13 vorteilhaft bezüglich Divergenz und Spotgröße für eine optimale Raman-Lichtausbeute ausgebildet sind.

Um die Sonde 2 möglichst kompakt auszubilden, kann es bei den zuvor beschriebenen Ausführungsbeispielen der optischen Sonde 2 vorteilhaft sein, die strahlformenden optischen Elemente 12, 19 als GRIN-Linsen mit ebenen Stirnflächen auszuführen. Weiterhin ist es vorteilhaft, die Umlenkelemente 13, 18 als Prismen auszuführen, wobei auch andere Ablenkwinkel als die gezeigten 90°-Winkel günstig sein können, um die optische Sonde 2 möglichst kompakt aufzubauen. Konstruktiv kann es auch vorteilhaft sein, den Strahlteiler-Filter 15 als Prismenstrahlteilerwürfel auszuführen, wobei auch hier Abweichungen von der 90°/45° Geometrie sinnvoll sein können, um das Schichtdesign des Strahlteiler-Filters 15 zu vereinfachen. Darüber hinaus kann die optische Sonde 2 vereinfacht werden, wenn die Filter 14, 16 auf Oberflächen der Linsenelemente 3, 4 oder der Umlenkelement 13, 18 als Beschichtung aufgebracht werden.

Die Erfindung ist nicht durch die Beschreibung anhand der Ausführungsbeispiele beschränkt. Die Erfindung ist in den unabhängigen Ansprüchen definiert. Vorteilhafte Weiterbildungen der Erfindung sind in den abhängigen Ansprüchen angegeben.

### Bezugszeichenliste

- 1: Objekt
- 2: Sonde
- 3: erstes Linsenelement
- 4: zweites Linsenelement
- 5: Bildebene
- 6: innerer Faserkern
- 7: äußerer Faserkern
- 8: Mantel
- 9: erste Lichtleitfaser
- 10: Faserscanner
- 11: zweite Lichtleitfaser
- 12: Strahlformungselement
- 13: Umlenkelement
- 14: Filter
- 15: Strahlteiler-Filter
- 16: Filter
- 17: optisches Element
- 18: Umlenkelement
- 19: strahlformendes optisches Element
- 20: Doublet-Linse
- 21: Plankonvex-Linse
- 22: Plankonvex- oder Plankonkavlinse
- 23: Frontfenster
- 24: Plankonvex-Linse
- 25: Meniskuslinse
- 26: Doublet-Linse
- 27: Doublet-Linse
- 28: rückseitiges Sondenende
- 29: frontseitiges Sondenende
- 30: Steuervorrichtung
- 31: Beleuchtungslicht
- 32: Objektlicht
- 33: Faserverbindung

## Patentansprüche

1. Optische Sonde (2), umfassend
- ein rückseitiges Ende (28) zum Anschluss an eine Auswerteeinheit (30) und ein frontseitiges Ende (29) zur optischen Untersuchung eines Objekts (1),
- einen ersten optischen Strahlengang für ein scannendes bildgebendes Verfahren und einen zweiten optischen Strahlengang für ein spektroskopisches Verfahren,
- eine erste Lichtleitfaser (9) in dem ersten optischen Strahlengang, die dazu eingerichtet ist, ein Beleuchtungslicht (31) für das scannende bildgebende Verfahren in Richtung des frontseitiges Endes (29) der Sonde (2) zu führen, und von dem Objekt (1) zurückkommendes Objektlicht (32) zum rückseitigen Ende (28) der Sonde (2) zu führen,
- eine Scanvorrichtung (10), die dazu eingerichtet ist, die erste Lichtleitfaser (9) oder das aus der ersten Lichtleitfaser (9) austretende Beleuchtungslicht (31) zum Abtasten des Objekts (1) bei dem scannenden bildgebenden Verfahren lateral abzulenken,
- eine zweite Lichtleitfaser (11) in dem zweiten optischen Strahlengang, die dazu eingerichtet ist, ein Anregungslicht oder ein detektiertes Objektlicht für das spektroskopische Verfahren zu führen, und
- einen Strahlteiler-Filter (15), wobei der erste optische Strahlengang des scannenden bildgebenden Verfahrens und der zweite optische Strahlengang des spektroskopischen Verfahrens mittels des Strahlteiler-Filters (15) in der Sonde (2) teilweise zur Überlappung gebracht werden, und wobei der Strahlteiler-Filter (15) den ersten Strahlengang und den zweiten Strahlengang in Richtung vom rückseitigen Ende (28) zum frontseitigen Ende (29) zusammenführt oder in umgekehrter Richtung voneinander trennt,
wobei die Sonde (2) einen Durchmesser von nicht mehr als 5 mm aufweist.

2. Optische Sonde nach Anspruch 1,
umfassend mindestens ein Linsenelement (3, 4, 17) zur Strahlformung des Beleuchtungslichts (31) und/oder des Objektlichts (32), wobei das mindestens eine Linsenelement (3, 4, 17) zumindest bereichsweise zwischen der ersten Lichtleitfaser (9) und dem frontseitigen Ende (29) der Sonde (2) angeordnet ist.

3. Optische Sonde nach einem der vorhergehenden Ansprüche, wobei die zweite Lichtleitfaser (11) dazu eingerichtet ist, das Anregungslicht des spektroskopischen Verfahrens zu führen, und die erste Lichtleitfaser (9) dazu eingerichtet ist, das detektierte Licht des spektroskopischen Verfahrens zu führen, oder wobei die erste Lichtleitfaser (9) dazu eingerichtet ist, das Anregungslicht des spektroskopischen Verfahrens zu führen, und die zweite Lichtleitfaser (11) dazu eingerichtet ist, das detektierte Licht des spektroskopischen Verfahrens zu führen.

4. Optische Sonde nach einem der vorhergehenden Ansprüche, wobei der Strahlteiler-Filter (15) wellenlängenselektiv ist und dazu eingerichtet ist, das Anregungslicht und das detektierte Licht des spektroskopischen Verfahrens voneinander zu trennen.

5. Optische Sonde nach einem der vorhergehenden Ansprüche, wobei der Strahlteiler-Filter (15) für das Beleuchtungslicht (31) und für das vom Objekt zurückkommende Objektlicht (32) des scannenden bildgebenden Verfahrens transmittierend ist.

6. Optische Sonde nach einem der vorhergehenden Ansprüche, wobei die Scanvorrichtung (10) ein Faserscanner oder ein Spiegelscanner ist.

7. Optische Sonde nach einem der vorhergehenden Ansprüche, wobei die erste Lichtleitfaser (9) einen inneren Faserkern (6) und einen äußeren Faserkern (7) aufweist, wobei der äußere Faserkern (7) einen Kerndurchmesser zwischen 20 µm und 300 µm und eine numerische Apertur zwischen 0,11 und 0,4 aufweist.

8. Optische Sonde nach einem der vorhergehenden Ansprüche, wobei die zweite Lichtleitfaser (11) einen Kerndurchmesser von 3 µm bis 300 µm und eine numerische Apertur von 0,08 bis 0,4 aufweist.

9. Optische Sonde nach einem der vorhergehenden Ansprüche, wobei die Sonde zumindest ein Linsenelement (4) aufweist, das zumindest eine Gradientenindexlinse umfasst.

10. Optische Sonde nach einem der vorhergehenden Ansprüche, wobei der zweite Strahlengang mindestens ein optisches Filter (14, 16) zur Selektion eines Wellenlängenbereichs für das spektroskopische Verfahren aufweist.

11. Optische Sonde nach einem der vorhergehenden Ansprüche, wobei das spektroskopische Verfahren Raman-Spektroskopie ist.

12. Verfahren zum Betrieb einer optischen Sonde nach einem der vorhergehenden Ansprüche,
bei dem ein Objekt (1) mittels der Sonde (2) mit mindestens einem scannenden bildgebenden Verfahren und mit mindestens einem spektroskopischen Verfahren untersucht wird.

13. Verfahren nach Anspruch 12,
wobei das scannende bildgebende Verfahren eines der folgenden Verfahren umfasst: optische Kohärenztomografie (OCT), CARS-Mikroskopie, SRS-Mikroskopie, Multiphotonenmikroskopie, SHG-Multiphotonenmikroskopie, konfokale Einzelphotonen-Fluoreszenz, Reflexionsmikroskopie.

14. Verfahren nach einem der Ansprüche 12 oder 13,
bei dem das spektroskopische Verfahren Raman-Spektroskopie ist.

## Claims

1. An optical probe (2) comprising
- a rear end (28) for connection to an evaluation unit (30) and a front end (29) for optically inspecting an object (1),
- a first optical beam path for a scanning imaging method and a second optical beam path for a spectroscopic method,
- a first optical fiber (9) in the first optical beam path configured to guide an illumination light (31) for the scanning imaging method toward the front end (29) of the probe (2) and to guide object light (32) returning from the object (1) to the rear end (28) of the probe (2),
- a scanning apparatus (10) configured to laterally deflect the first optical fiber (9) or the illumination light (31) emerging from the first optical fiber (9) for scanning the object (1) in the scanning imaging method,
- a second optical fiber (11) in the second optical beam path configured to guide an excitation light or a detected object light for the spectroscopic method, and
- a beam splitter filter (15), wherein the first optical beam path of the scanning imaging method and the second optical beam path of the spectroscopic method are partially brought to overlap by means of the beam splitter filter (15) in the probe (2), and wherein the beam splitter filter (15) merges the first beam path and the second beam path in the direction from the rear end (28) to the front end (29) or separates them from each other in the opposite direction,
wherein the probe (2) has a diameter of not more than 5 mm.

2. The optical probe (2) according to claim 1,
comprising at least one lens element (3, 4, 17) for beam shaping the illumination light (31) and/or the object light (32), wherein the at least one lens element (3, 4, 17) is arranged at least regionally between the first optical fiber (9) and the front end (29) of the probe (2).

3. The optical probe according to any of the preceding claims,
wherein the second optical fiber (11) is configured to guide the excitation light of the spectroscopic method, and the first optical fiber (9) is configured to guide the detected light of the spectroscopic method,
or wherein the first optical fiber (9) is configured to guide the excitation light of the spectroscopic method and the second optical fiber (11) is configured to guide the detected light of the spectroscopic method.

4. The optical probe according to any of the preceding claims,
wherein the beam splitter filter (15) is wavelength-selective and is configured to separate the excitation light and the detected light of the spectroscopic method from each other.

5. The optical probe according to any of the preceding claims,
wherein the beam splitter filter (15) is transmitting for the illumination light (31) and for the object light (32) returning from the object of the scanning imaging method.

6. The optical probe according to any of the preceding claims,
wherein the scanning device (10) is a fiber scanner or a mirror scanner.

7. The optical probe according to any of the preceding claims,
wherein the first optical fiber (9) comprises an inner fiber core (6) and an outer fiber core (7), the outer fiber core (7) having a core diameter between 20 µm and 300 µm and a numerical aperture between 0.11 and 0.4.

8. The optical probe according to any of the preceding claims,
wherein the second optical fiber (11) has a core diameter from 3 µm to 300 µm and a numerical aperture from 0.08 to 0.4.

9. The optical probe according to any of the preceding claims,
wherein the probe comprises at least one lens element (4) comprising at least one gradient index lens.

10. The optical probe according to any of the preceding claims,
wherein the second beam path comprises at least one optical filter (14, 16) for selecting a wavelength range for the spectroscopic method.

11. The optical probe according to any of the preceding claims,
wherein the spectroscopic method is Raman spectroscopy.

12. A method for operating an optical probe according to one of the preceding claims,
wherein an object (1) is examined by means of the probe (2) with at least one scanning imaging method and with at least one spectroscopic method.

13. The method according to claim 12,
wherein the scanning imaging method comprises one of the following methods: optical coherence tomography (OCT), CARS microscopy, SRS microscopy, multiphoton microscopy, SHG multiphoton microscopy, confocal single photon fluorescence, reflection microscopy.

14. The method according to claim 12 or 13,
wherein the spectroscopic method is Raman spectroscopy.

## Revendications

1. Sonde optique (2) comportant :
- une extrémité arrière (28) en vue de la connexion à une unité d'évaluation (30) et une extrémité frontale (29) en vue de l'examen optique d'un objet (1),
- une première trajectoire optique du faisceau destinée à un procédé d'imagerie par balayage et une seconde trajectoire optique du faisceau destinée à un procédé spectroscopique,
- une première fibre optique (9) dans la première trajectoire optique du faisceau, qui est installée afin de guider une lumière d'éclairage (31) destinée au procédé d'imagerie par balayage dans la direction de l'extrémité frontale (29) de la sonde (2), et afin de guider la lumière d'objet (32) revenant de l'objet (1) vers l'extrémité arrière (28) de la sonde (2),
- un dispositif de balayage (10) qui est installé afin de dévier latéralement la première fibre optique (9) ou la lumière d'éclairage (31) sortant de la première fibre optique (9) en vue du balayage de l'objet (1) lors du procédé d'imagerie par balayage,
- une seconde fibre optique (9) dans la seconde trajectoire optique du faisceau, qui est installée afin de guider une lumière d'excitation ou une lumière d'objet détectée en vue du procédé spectroscopique, et
- un filtre de séparateur de faisceaux (15), sachant que la première trajectoire optique du faisceau du procédé d'imagerie par balayage et la seconde trajectoire optique du faisceau du procédé spectroscopique sont amenées partiellement à se chevaucher au moyen du filtre de séparateur de faisceaux (15) dans la sonde (2), et sachant que le filtre de séparateur de faisceau (15) réunit la première trajectoire du faisceau et la seconde trajectoire du faisceau dans la direction de l'extrémité arrière (28) vers l'extrémité frontale (29) ou les sépare l'une de l'autre dans la direction inverse,
sachant que la sonde (2) présente un diamètre non supérieur à 5 mm.

2. Sonde optique selon la revendication 1,
comportant au moins un élément de lentille (3, 4, 17) en vue de la formation de faisceau de la lumière d'éclairage (31) et/ou de la lumière d'objet (32), sachant qu'au moins l'un des éléments de lentille (3, 4, 17) est disposé au moins par zones entre la première fibre optique (9) et l'extrémité frontale (29) de la sonde (2).

3. Sonde optique selon une quelconque des revendications précédentes,
sachant que la seconde fibre optique (11) est installée pour guider la lumière d'excitation du procédé spectroscopique, et la première fibre optique (9) est installée pour transporter la lumière détectée du procédé spectroscopique,
ou sachant que la première fibre optique (9) est installée pour guider la lumière d'excitation du procédé spectroscopique, et la première fibre optique (11) est installée pour transporter la lumière détectée du procédé spectroscopique.

4. Sonde optique selon une quelconque des revendications précédentes,
sachant que le filtre de séparateur de faisceau (15) est sélectif en longueur d'onde et est installé afin séparer l'une de l'autre la lumière d'excitation et la lumière détectée de la méthode spectroscopique.

5. Sonde optique selon une quelconque des revendications précédentes,
sachant que le filtre de séparateur de faisceau (15) est transmetteur pour la lumière d'éclairage (31) et pour la lumière d'objet (32) du procédé d'imagerie par balayage revenant de l'objet.

6. Sonde optique selon une quelconque des revendications précédentes,
sachant que le dispositif de balayage (10) est un scanneur à fibres ou un scanneur à miroir.

7. Sonde optique selon une quelconque des revendications précédentes,
sachant que la première fibre optique (9) présente un noyau de fibre interne (6) et un noyau de fibre externe (7), sachant que le noyau de fibre externe (7) présente un diamètre de noyau compris entre 20 µm et 300 µm et une ouverture numérique comprise entre 0,11 et 0,4.

8. Sonde optique selon une quelconque des revendications précédentes,
sachant que la seconde fibre optique (11) présente un diamètre de noyau de 3 µm à 300 µm et une ouverture numérique de 0,08 à 0,4.

9. Sonde optique selon une quelconque des revendications précédentes,
sachant que la sonde présente au moins un élément de lentille (4) qui comporte au moins une lentille à indice de gradient.

10. Sonde optique selon une quelconque des revendications précédentes,
sachant que la seconde trajectoire du faisceau présente au moins un filtre optique (14, 16) en vue de la sélection d'un domaine spectral destinée au procédé spectroscopique.

11. Sonde optique selon une quelconque des revendications précédentes,
sachant que le procédé spectroscopique est une spectroscopie Raman.

12. Procédé en vue du fonctionnement d'une sonde optique selon une quelconque des revendications précédentes, lors duquel un objet (1) est examiné au moyen de la sonde (2) avec au moins un procédé d'imagerie par balayage et avec au moins un procédé spectroscopique.

13. Procédé selon la revendication 12,
sachant que le procédé d'imagerie par balayage comporte un des procédés suivants : la tomographie par cohérence optique (OCT), la microscopie CARS, la microscopie SRS, la microscopie multiphotonique, la microscopie multiphotonique SHG, la fluorescence monophotonique confocale, la microscopie électronique par bombardement.

14. Procédé selon une quelconque des revendications 12 ou 13,
sachant que le procédé spectroscopique est une spectroscopie Raman.
